# EUROPEAN PATENT APPLICATION

(11) **EP 2 915 689 A1**
(43) Date of publication of application: **09.09.2015**
(21) Application number: 15000613.8
(22) Date of filing: 03.03.2015
(51) Int. Cl.: B60K 26/02, B60T 7/06, B60T 7/08, B60T 7/10, G05G 1/487, G05G 1/54, B60W 10/00, B60W 40/08

(54) **Pedal control**

(30) Priority: 03.03.2014 GB 201403668
(71) Applicant: Short, Peter, Westcott HP18 0JX (GB)
(72) Inventor: Short, Peter, Westcott HP18 0JX (GB)
(74) Representative: Dutton, Erica L. G.

(57) **Abstract**

In a push-pull brake and accelerator control, the brake operating assembly has first and second rods with a pivoting attachment between their ends. The rods extend between the brake pedal of a standard vehicle to an operating handle near the steering wheel. The lower end of the first rod sits forward of the brake pedal, towards the front of the vehicle, and the second rod extends at an angle to the first rod, from its lower end to a pivotal pedal fixing to the brake pedal. The pivoting attachment between the rods has a stop on it so that the two rods can be brought to a minimum angle between them. Movement of the first rod away from the brake pedal increases the angle and no force is exerted on the brake pedal. Movement in the other direction brings the two rods to the minimum angle, whereafter the two rods move as a single unit and bring pressure to bear on the brake pedal.

## Description

The present invention relates to a pedal control for a vehicle and finds particular application as a hand-operated pedal control, for instance for a brake and/or accelerator pedal.

It is known to use a telescopic rod in a "push-pull" hand control that is used for both the brake pedal and the accelerator of a vehicle. The rod extends from the brake pedal towards the steering column. The top of the rod has an operating handle near the steering wheel and the bottom of it is attached to a depressible part of the brake pedal. A first movement of the handle telescopes the rod until it exerts a force on the brake pedal. A second movement of the handle opens out the telescopic rod. This has no effect on the brake pedal because the bottom of the rod isn't subject to a force but a cable attached to the pulled section of the rod, the upper part, acts through a pivoting attachment to depress the accelerator pedal.

These "push-pull" hand controls can be used to modify the operation of conventional brake and accelerator pedals without modifying the pedals themselves. Hence the pedals can still be conventionally foot-operated.

According to embodiments of the present invention in a first aspect, there is provided a hand control for a pedal of a vehicle, the hand control comprising:
i) a first rod having a pivotal attachment to a second rod;
ii) a pivotal pedal fixing for fixing the second rod to the pedal; and
iii) a stop on the pivotal attachment so that the second rod reaches a stopped position in which it is stopped from pivotting relative to the first rod through an angular range less than a predetermined stop angle.

The hand control may further comprise a bearing sleeve for mounting it in the vehicle. The first rod, being provided with an operating device such as a handle, can be mounted in the bearing sleeve for bi-directional movement in response to the operating device. With the second rod being attached to the pedal by means of the pivotal pedal fixing, the stopped pivotal attachment between the first and second rods then allows the operating device:
- in a first movement, to pull the first rod so as to increase the angle between the two rods without moving the pedal, and
- in a second movement, to push the first rod so that the second rod reaches the stopped position and then pushes on the pedal.

An advantage of the pivotal attachment between the two rods is that the first rod can be installed in a better position in relation to the cabin of a vehicle compared to the known telescoping rod described above. Linear travel of the telescoping rod acts directly onto the pedal. This limits the positioning of the telescoping rod and can mean for example that knee airbags have to be disconnected as the rod has to run below the airbag. In embodiments of the invention in its first aspect, the hand control can be mounted higher up, or further forward, into a steering cowling, giving more clearance for the driver. Also the first rod can be routed above or forward of knee airbags and above the lower dash panel, this enabling the knee airbags to remain operative.

Although not essential, the stop angle may be at least approximately a right angle because this allows the maximum displacement upwards and/or forwards of the lower end of the first rod in the vehicle cabin compared with the positioning of operative rods in known hand controls. The stop angle might for example be in the range from 45° to 135° and preferably in the range from 80° to 100°.

The movement of a vehicle's pedal, such as the brake pedal, is usually pivotal but the bearing sleeve in its simplest form might provide a straight channel so that the movement of the first rod is linear. When the pedal is depressed, its pivotal movement will cause a change in distance between the pivotal attachment between the two rods and the pivotal pedal fixing. This can be accommodated for example by using a telescopic second rod, or a pivotal pedal fixing which also offers a sliding movement of the second rod relative to the pedal.

To provide a simple push/pull operation of the hand control which will operate both a brake pedal and an accelerator, the first rod can be provided with a cable attaching it to a pivoting pedal actuator, the actuator being mountable to the vehicle to provide a fulcrum partway along its length, such that tension on the cable brings the actuator to bear on the second pedal. Thus pulling the hand control depresses the second pedal via the actuator, for instance the accelerator, while pushing the hand control depresses the first pedal, for instance the brake, via the pivotal pedal fixing.

A brake control according to embodiments of the present invention will now be described, by way of example only, with reference to the following drawings in which:
Figure 1 shows schematically, in side elevation, the brake control installed in a vehicle;
Figure 2 shows, in side elevation, a view of a pivotal pedal fixing between the brake control and a brake pedal;
Figure 3 shows a plan view of the fixing of Figure 2;
Figures 4a and 4b show, in side elevation, the brake control being pulled in a first movement which doesn't move the brake pedal; and
Figure 5 shows, in side elevation, the brake control being pushed in a second movement which depresses the brake pedal.

It should be noted that the figures are schematic only and not drawn to scale.

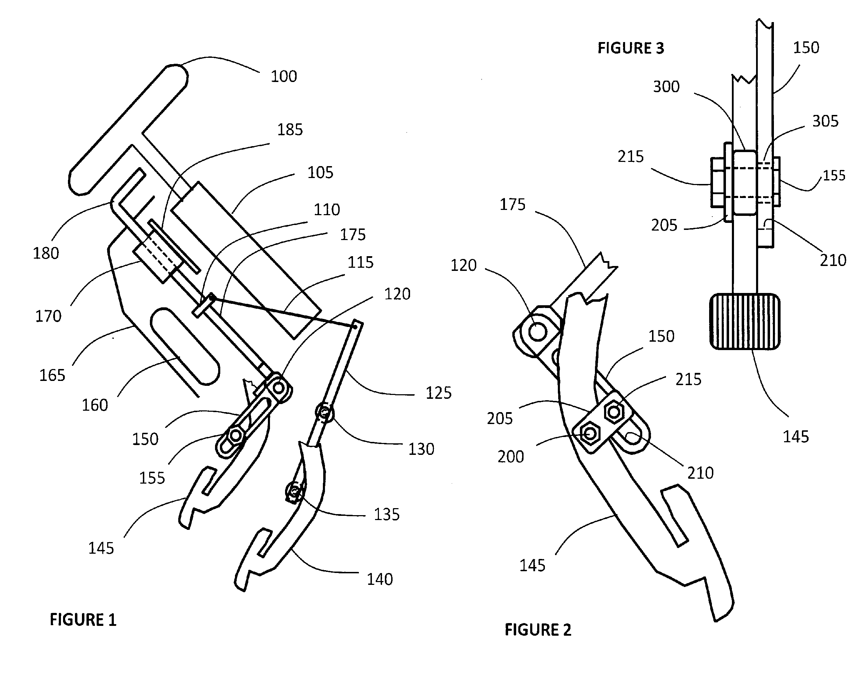

Referring to Figure 1, the brake control has an operating handle 180 attached to a first rod 175 which in turn is attached to a second rod 150. The first rod 175 is mounted for longitudinal movement in a bearing sleeve 170 attached by a plate 185 to the vehicle cabin. The second rod 150 is attached to the brake pedal 145. The first and second rods 175, 150 have a pivotal attachment 120 between them. The second rod 150 has a sliding/pivoting attachment via a bolt 155 to the brake pedal 145.

The operating handle 180 is adjacent the steering wheel 100 of the vehicle and the first rod 175 extends roughly parallel to the steering column 105. A knee airbag 160 is mounted rearward of the first rod 175 so that it can inflate to protect a driver. A dash panel 165 largely conceals the airbag 160 and first rod 175 from a driver.

The operating handle 180 can be used to operate an accelerator pedal 140 of the vehicle via a pivoting pedal actuator in known manner. The pivoting pedal actuator is primarily a lever 125 pivotally mounted to the vehicle. A cable 115 runs from a mounting point 110 on the first rod 175 to a cable attachment end of the lever 125 which operates the accelerator pedal 140 when the operating handle 180 is pulled sufficiently upwards. The lever 125 has a pivotal mounting 130 to the vehicle, providing a fulcrum when the cable 115 pulls on the cable attachment end. An accelerator-operating end of the lever 125, beyond the fulcrum from the cable attachment end, depresses the accelerator via a roller 135 when the operating handle 180 is pulled sufficiently upwards.

Connection of the operating handle 180 for operation of the brake pedal 145 is now described. Referring to Figures 2 and 3, the attachment of the second rod 150 to the brake pedal 145 is via a pivotal pedal fixing which offers both a sliding and a pivoting action. The second rod 150 has a slot 210 in it and a bolt 215 fixed to the brake pedal 145 extends through the slot 210, being slidably secured there by a nut 155. This sliding bolt 215 is fixed above the shaft of the brake pedal 145 with a plate and bolt assembly 205, 200. The plate 205 is fixed to the shaft of the brake pedal 145 with a fixing bolt 200 and extends upwardly to carry the sliding bolt 215. The sliding bolt 215 extends over the top of the shaft of the brake pedal 145, resting on the shaft via a resilient pad 300, and into the slot 210. Where the bolt 215 seats in the slot 210, it carries a bearing ring 305 to prevent wear where the bolt 215 both rotates and travels linearly in the slot 210. Thus the pivotal pedal fixing has several components, the plate and bolt assembly 205, 200, the bolt and nut 215, 155 fixing the second rod 150 to the plate and bolt assembly 205, 200 and the resilient pad 300. The bolt 215 acts as a projection of the pivotal pedal fixing which runs in the slot 120 in operation of the brake pedal 145.

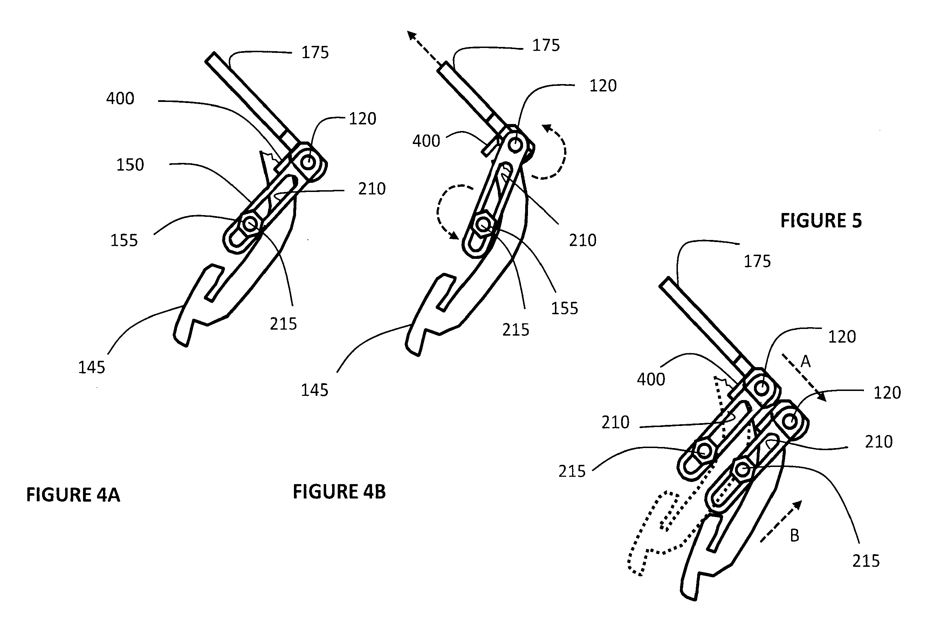

Referring to Figures 4a and 4b, when the operating handle 180 is used to operate the accelerator 140, there is no effect on the brake pedal 145. This is because when the handle 180 is pulled upwards, the angle between the first and second rods 175, 150 simply opens up. The pivotal attachment 120 between them opens up and the second rod 150 pivots towards its other end about the sliding bolt 215. This double pivoting action is indicated by the dotted arrows in Figure 4b.

Referring additionally to Figure 5, when the operating handle is used to operate the brake 145 however, the brake pedal 145 is depressed. This is the result of a stop 400 on the pivotal attachment 120 between the two rods 175, 150. The stop 400 prevents the angle between the two rods 175, 150 decreasing below a right angle. Hence when the handle 180 is pushed downwards to the point where the angle between the two rods 175, 150 is a right angle, no more pivoting will occur. Further pressure on the handle 180 now acts to move the whole second rod 150 in a direction parallel to the axial direction of the first rod 175, indicated by the dotted arrow "A". This applies braking pressure to the pedal 145 via the sliding bolt 215.

The slot 120 and the sliding bolt 215 together provide a sliding mechanism to accommodate movement of the brake pedal 145 when depressed. Where the brake pedal 145 has a pivoting rather than a linear action, the linear movement of the second rod 150 after it reaches the stop 400 produces a change in position of the sliding bolt 215 along the slot 210 in the second rod 150, in a direction indicated by the dotted arrow "B".

Known brake pedals 145 in vehicles have this pivoting action. However, if a vehicle has a linearly operating brake pedal 145, either the first rod 175 can be arranged to move in a direction parallel to the movement of the brake pedal 145 when braking, or a sliding mechanism for the second rod 150 as described above will also accommodate non-parallel movement.

Alternative arrangements for facilitating the relative movements of the second rod 150 and the brake pedal 145 during braking can be made. Instead of the slot and bolt arrangement 210, 215 described above, it would be possible for example to have a purely pivotal attachment between the second rod 150 and the brake pedal 145 and to accommodate the change in separation between the end of the first rod 175 and that pivotal attachment by using a telescopic second rod 150. The stop 400 would still act so that the handle 180 moves the second rod 150 as described above, during braking, and thus depress the brake pedal 145. However, the slotted arrangement as described above may be found more robust.

The stop 400 is shown as acting on the upper surface of the second rod 150 but could be designed for instance to be internal to the pivotal attachment 120 between the two rods 175, 150. This would prevent debris getting between the stop 400 and the second rod 150.

The stop 400 is described above as limiting the angle between the two rods 175, 150 so that it doesn't go below a right angle. This is not essential. It is only essential that the angle has a lower limit, beyond which the second rod 150 is moved as a whole by pressure on the operating handle 180. The lower limit might be determined by a required positioning of the two rods 175, 150 in the vehicle, for instance to avoid the airbag 160. Where the lower limit is a right angle, this allows the first rod 175 to be positioned as far forward as possible at the pivotal attachment 120 and therefore as well clear as possible of the airbag 160 while minimising the length of the second rod 150. For long term operation of the stop 400, it might be preferred however that the lower limit is greater or less than a right angle, for instance in the range 45° to 135°, to reduce the turning moment experienced by the stop 400 during braking. The stop 400 itself might be differently shaped, being provided as a block or other obstruction to the pivotal attachment 120.

The nature of the operating handle 180 is not critical. It might be represented by a less direct mechanism to act on the first rod 175, such as a lever, or a button operating a solenoid. However, the two-part assembly as described above, particularly where the handle 180 is part of the first rod 175 and the first rod 175 brakes the vehicle via just the second rod 150, offers simplicity and strength and still accommodates an operative, standard knee airbag 160.

The two rods 175, 150 are shown as straight but this isn't essential. It is convenient that the first rod 175 has a straight section that travels in the bearing sleeve 170 during operation but the bearing sleeve 170 could alternatively be curved and a section of the first rod 175 could also be curved so as to travel in it.

## Claims

1. A hand control for a pedal of a vehicle, the hand control comprising:
iv) a first rod having a pivotal attachment to a second rod;
v) a pivotal pedal fixing for fixing the second rod to the pedal; and
vi) a stop on the pivotal attachment so that the second rod reaches a stopped position in which it is stopped from pivotting relative to the first rod through an angular range less than a predetermined stop angle.

2. A hand control according to claim 1 wherein the stop angle is in the range from 45° to 135° and preferably in the range from 80° to 100°.

3. A hand control according to either preceding claim, wherein the first rod is provided with a bearing sleeve for mounting it in the vehicle.

4. A hand control according to claim 3, having an operating device fixed to the first rod, the hand control being mounted in a vehicle by means of the bearing sleeve and the second rod being attached to the pedal by means of the pivotal pedal fixing, such that the pivotal attachment between the first and second rods allows the operating device:
• in a first operation, to move the first rod away from the pedal so as to increase the angle between the two rods without moving the pedal, and
• in a second operation, to move the first rod towards the pedal so that the second rod reaches the stopped position and thereafter depresses the pedal.

5. A hand control according to any preceding claim, wherein the first rod is mounted clear of at least one operative knee airbag in the vehicle.

6. A hand control according to any preceding claim, wherein the vehicle has at least one operative knee airbag and a steering device and wherein the first rod is provided with an operating handle, the first rod being mounted above or forward of the airbag in the vehicle and the operating handle being positioned adjacent to the steering device.

7. A hand control according to any preceding claim, wherein the second rod is provided with a sliding mechanism for accommodating a change in separation between the pivotal pedal fixing and the pivotal attachment between the first and second rods during use.

8. A hand control according to claim 7 wherein the second rod has a telescopic action providing the sliding mechanism.

9. A hand control according to claim 7 wherein the second rod has a slot accommodating a projection of the pivotal pedal fixing to provide the sliding mechanism.

10. A hand control according to any one of the preceding claims, for use in operating first and second pedals, the first rod being provided with a cable to attach it to a pivoting pedal actuator, the actuator being mountable to the vehicle to provide a fulcrum partway along its length, such that tension on the cable brings the actuator to bear on the second pedal.

11. A hand control according to claims 4 and 10, the vehicle having brake and accelerator pedals, wherein the actuator is mounted to the vehicle such that, in the first operation the actuator bears on the accelerator pedal and, in the second operation, the second rod depresses the brake pedal.

12. A vehicle comprising a hand control according to any one of the preceding claims.

13. A vehicle according to claim 12, the vehicle having at least one operative knee airbag and wherein the first rod is routed above or forward of the airbag..
